# EUROPEAN PATENT SPECIFICATION

(11) **EP 3 852 620 B1**
(45) Date of publication and mention of the grant of the patent: **25.10.2023**
(21) Application number: 19790064.0
(22) Date of filing: 19.09.2019
(51) Int. Cl.: A61B 5/03, A61B 5/06, A61M 16/00, A61J 15/00, A61B 5/00

(54) **NASOGASTRIC PROBE**
NASOGASTRISCHE SONDE
SONDE NASOGASTRIQUE

(30) Priority: 21.09.2018 IT 201800008797
(43) Date of publication of application: 28.07.2021
(73) Proprietor: Eurosets S.r.l., 41036 Medolla (MO) (IT)
(72) Inventor: PETRALIA, Antonio, 41036 Medolla (MO) (IT); GHELLI, Nicola, 41036 Medolla (MO) (IT); FONTANILI, Paolo, 41036 Medolla (MO) (IT)
(74) Representative: Zoli, Filippo
(86) International application number: PCT/IB2019/057928
(87) International publication number: WO 2020/058909

(56) References cited:
- EP-A1- 3 103 430
- CN-A- 106 691 866
- CN-U- 203 107 729
- CN-U- 204 864 127
- KR-A- 20090 090 667
- US-A1- 2014 235 960
- US-A1- 2017 143 932

## Description

### Technical Field

The present invention relates to a nasogastric probe.

### Background Art

In the medical field it has long been known that nasogastric probes are used for various purposes such as, e.g., sucking up the gastric contents, performing enteral nutrition, emptying the stomach of dangerous contents (e.g. stomach pumping or gastric lavage), preventing the relaxation of the stomach before or after surgery.

It is also known to perform enteral nutrition on artificially ventilated patients, for example when the normal vital functions of a patient are impaired or altered as a result of an acute disease or a traumatic event.

The known type of nasogastric probes generally comprises a tubular element of elongated shape and flexible type, of the type of silicone or polyurethane, of variable section and length depending on the type of application and the age of the patient.

The tubular element is provided at one end with at least one feeding mouth for the introduction of nutritive substances or drugs and at the opposite end with one or more delivery mouths, where the feeding mouth and the delivery mouths are connected to each other by means of a main channel.

Some probes of known type are provided with means for detecting the pressure inside the patient's oesophagus, in particular in order to adequately regulate the patient's assisted ventilation.

These pressure detecting means are of the type of an inflatable balloon connected to a corresponding transducer, placed outside the patient, capable of determining the pressure which is on the outside of the balloon itself.

These probes of known type do however have some drawbacks.

They do not allow in fact a precise and punctual detection of the pressure inside the patient's esophagus, since this detection depends on the deformation of the balloon, which is also appropriate to be in contact with the walls of the esophagus itself to perform its function properly.

The probes of known type do not thus allow detecting any barotrauma that may occur as a result of compression of the oesophagus by the lungs due to the air introduced into them through forced ventilation, with a consequent risk for the patient.

In addition, the probes of known type do not allow the medical operator to promptly and effectively identify any incorrect insertion by the medical staff, e.g. inside the respiratory tract.

Today, to verify the correct placement of the nasogastric probes, various methods exist: air can be blown into the patient's stomach and, by means of a phonendoscope positioned on the patient's stomach, the noises created by the passage of air can be heard, an X-ray check can be carried out, or probes provided with a magnetic element can be used to monitor the position thereof. These methods are not however very practical to use, they are not always reliable and, in particular, radiographic monitoring exposes the patient to the risk of radiation. US 2014235960 discloses a nasogastric probe according to the preamble of the claim 1.

### Description of the Invention

The main aim of the present invention is to devise a nasogastric probe that allows detecting in a practical and effective manner the pressure inside the esophagus after the probe itself has been inserted inside the patient.

Within this aim, one object of the present invention is to allow a prompt and effective regulation of the forced ventilation of the patient.

Another object is to devise a probe that allows easy identification of its position, so as to easily identify any incorrect insertion into the airway.

Another object of the present invention is to devise a nasogastric probe that allows overcoming the aforementioned drawbacks of the prior art in a simple, rational, easy, effective to use and low cost solution.

The aforementioned objects are achieved by the present nasogastric probe according to claim 1.

### Brief Description of the Drawings

Other characteristics and advantages of the present invention will be more evident from the description of a preferred, but not exclusive, embodiment of a nasogastric probe, illustrated as an indication, but not limited to, in the attached tables of drawings in which:

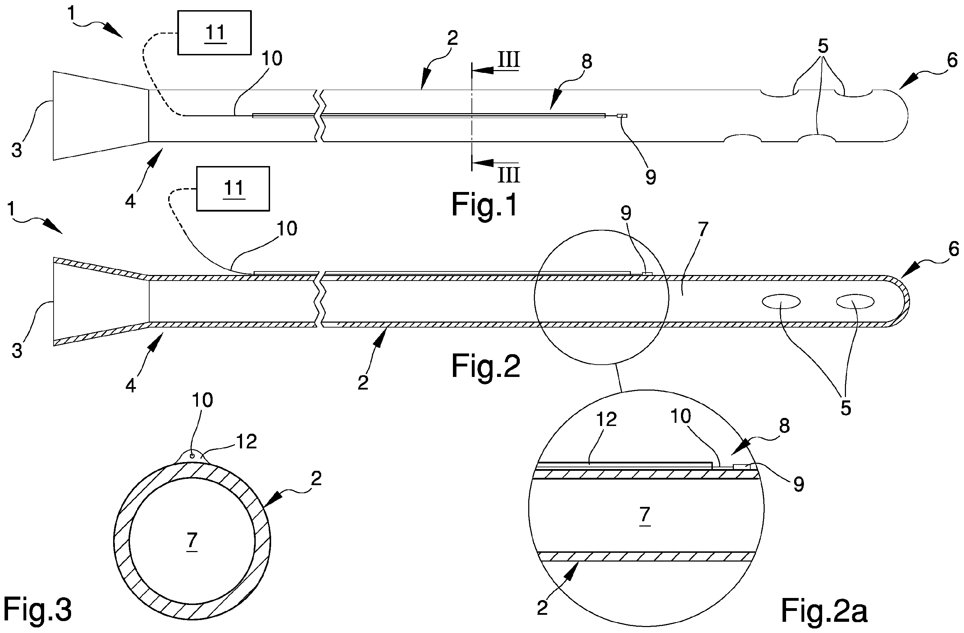

Figure 1 is a schematic view in lateral elevation of a probe according to the invention;
Figure 2 is a cross-sectional view along a longitudinal plane of the probe in Figure 1;
Figure 2a is a magnification of a detail of the probe in Figure 2;
Figure 3 is a cross-sectional view along a transverse plane of the probe in Figure 1;
Figure 4 is a schematic representation of a piece of equipment according to the invention.

### Embodiments of the Invention

With particular reference to these figures, reference numeral 1 globally indicates a nasogastric probe.

The probe 1 comprises at least one substantially flexible tubular element 2 of elongated shape, made e.g. of silicone or polyurethane.

More particularly, the cross section and length of the tubular element 2 may vary depending on the type of application and the age of the patient.

The tubular element 2 is provided with at least one feeding mouth 3 for the administration of nutritive substances, drugs or the like, which is arranged at a first end stretch 4 of the tubular element itself, and with one or more delivery mouths 5 of the substances introduced through the feeding mouth 3, located at a second end stretch 6 opposite the first end stretch 4 and intended to be inserted into the patient's stomach passing through the esophagus.

The feeding mouth 3 and the delivery mouths 5 are placed in communication with each other by means of a main channel 7 which crosses the tubular element 2 longitudinally.

The probe 1 also comprises pressure detecting means 8 at the point where the patient's esophagus is located.

According to the invention, the pressure detecting means 8 comprise at least one pressure sensor 9 associated with the tubular element 2 and placed between the feeding mouth 3 and the delivery mouths 5. In particular, the pressure sensor 9 is placed between the feeding mouth 3 and the delivery mouth 5 closest to the feeding mouth itself.

The pressure sensor 9 is then placed on top of the delivery mouths 5.

Advantageously, the pressure sensor 9 is arranged at a distance from the feeding mouth 3 equal to about 2/3 of the length of the tubular element 2 (a margin of tolerance is provided to allow placement within the esophagus after the probe 1 has been correctly inserted in the patient).

The pressure sensor 9 is according to the invention arranged externally to the main channel 7, so it does not interfere with the passage of fluids or substances inside it. More in detail, according to the invention and as shown in the figures, the pressure sensor 9 is associated with the external surface of the tubular element 2.

According to the invention, the pressure sensor 9 is the type of a pressure transducer, thus adapted to convert a pressure signal into an electrical signal.

The pressure transducer 9 is fixed e.g. to the tubular element 2 by means of gluing means such as, e.g., of the type of a resin.

In a particular embodiment, not shown in the figures, the pressure detecting means 8 comprise a plurality of pressure sensors 9 arranged mutually spaced apart along the longitudinal extension of the tubular element 2 in order to detect the pressure inside the patient in a plurality of points which are separate from each other.

The pressure detecting means 8 therefore also comprise electric connection means 10 of the pressure sensor 9 to pressure reading means 11, of the type of a monitor or the like.

The connection means 10 can therefore be, e.g., of the type of electrical cables or of the type of an optical fiber.

Appropriately, the tubular element 2 also comprises one connection channel 12, separate from the main channel 7 and adapted to contain the connection means 10.

More particularly, the connection channel 12 is defined at the outer wall of the tubular element 2 which delimits the main channel 7. In the embodiment shown in the figures, the connection channel 12 extends substantially parallel to the main channel 7.

It should be noticed that the diameter of the connection channel 12 is significantly smaller than that of the main channel 7; therefore, the presence of the connection channel 12 does not substantially alter the overall dimensions of the tubular element 2 and therefore does not adversely affect the method of insertion into the patient's body.

The pressure sensor 9 is advantageously arranged externally to the connection channel 12 so that it is immersed in the environment where the tubular element 2 is located.

Alternative embodiments, not according to the invention, cannot however be ruled out wherein the pressure sensor 9 is arranged internally to the main channel 7. In this alternative embodiment, not shown in the figures, the connection channel 12 and the connection means 10 are arranged externally to the main channel 7 and the wall of the tubular element 2 which delimits the main channel 7 has a through hole for the insertion of the pressure sensor 9 internally to the main channel itself.

In a particular embodiment, not shown in the figures, the probe 1 comprises an additional pressure sensor associated with the tubular element 2 at the second end stretch 6 in order to detect the pressure at the delivery mouths 5.

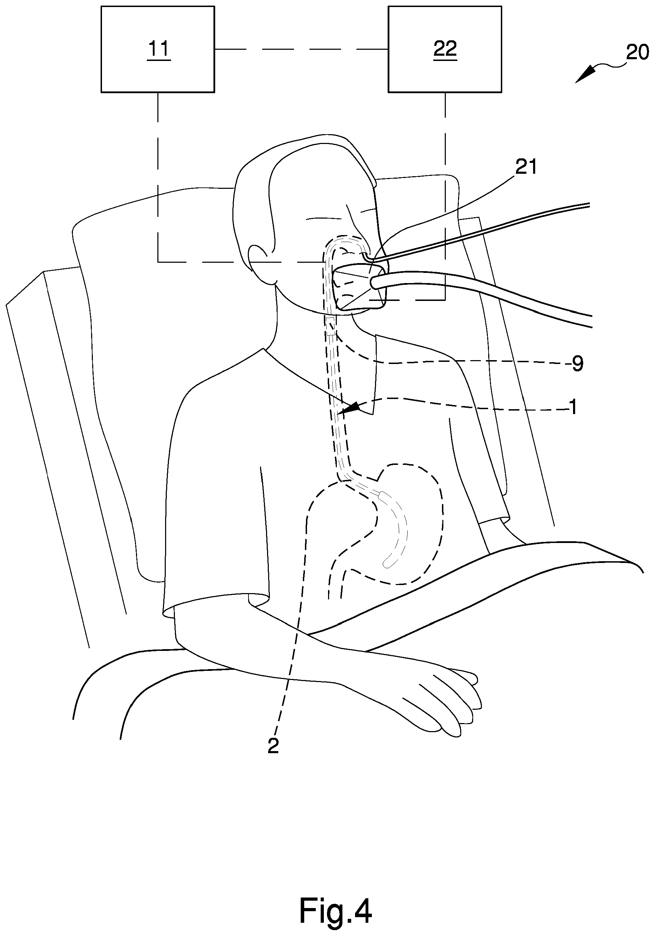

The present invention also relates to a piece of equipment, identified in Figure 4 with reference numeral 20, for the forced breathing of a patient, comprising the probe 1 according to what has been described above, assisted ventilation means 21, of the type of a lung ventilator that can be connected to the respiratory apparatus of a patient, and adapted to convey a working gas (e.g. air and oxygen or oxygen only) and at least one control and command unit 22 operatively connected to the ventilation means 21 and to the probe 1, where this control and command unit 22 is programmed to regulate the conveyed amount of working gas according to the pressure detected by the pressure sensor 9.

The control and command unit 22, of the type of an electronic control unit provided with a microprocessor, is preferably programmed to reduce the amount of the working gas delivered by the ventilation means 21 when the pressure detected by the pressure sensor 9 exceeds a predefined value.

The operation of the present invention is as follows.

The probe 1 is inserted into the patient's digestive system utilizing the common methods used today. Following the insertion into the patient, the pressure sensor 9 is positioned along the esophagus, thus allowing almost continuous monitoring of the pressure inside the esophagus.

The data provided by the pressure sensor 9 and read by the medical staff through the reading means 11 allow monitoring the pressure inside the esophagus in order to verify the presence of any overpressure exerted by the lungs and due to the forced ventilation applied to the patient. The pressure values "read" by the pressure sensor 9 may indicate excessive or insufficient ventilation. For example, if the detected pressure exceeds a predefined reference value, it is likely that the lung ventilator 21 connected to the patient is providing an excess of air compared to the patient's real needs, who may then experience lung trauma. The control and command unit 22 then intervenes retroactively on the lung ventilator 21, e.g. by reducing the amount of air it introduces into the patient's lungs.

The pressure values provided by the pressure sensor 9 also allow medical staff to understand if the probe itself has been correctly inserted into the digestive system or if it has been incorrectly introduced along the airway, so it must be extracted and perform the operation again.

It has in practice been ascertained that the described invention achieves the intended objects and, in particular, it is underlined that the probe, to which the present invention relates, allows the pressure inside the patient's esophagus to be detected in a simple and effective way.

The probe according to the invention thus allows medical staff to identify promptly the risk of barotrauma and to regulate the forced ventilation of the patient in a practical and effective way.

In addition, the probe in question allows quickly identifying any incorrect insertion of the probe itself into the respiratory apparatus.

## Claims

1. Nasogastric probe (1), comprising:
- at least one substantially flexible tubular element (2) of elongated shape, provided with at least one main channel (7) communicating with at least one feeding mouth (3) of nutritive substances or the like, which is arranged at a first end stretch (4) of the tubular element itself, and with at least one delivery mouth (5) of nutritive substances or the like, which is arranged at a second end stretch (6) opposed to said first end stretch (4) and intended to be inserted into the patient's stomach passing through the esophagus;
- pressure detecting means (8) comprising at least one pressure sensor (9) associated with said tubular element (2) and interposed between said feeding mouth (3) and said delivery mouth (5), where said pressure sensor (9) is the type of a pressure transducer, thus adapted to convert a pressure signal into an electrical signal;
**characterized by** the fact that said pressure sensor (9) is arranged externally to said main channel (7), so it does not interfere with the passage of fluids or substances inside it, and it is associated with the external surface of said tubular element (2), and is configured to detect overpressure from the lungs and caused by forced ventilation.

2. Probe (1) according to claim 1, **characterized by** the fact that said pressure sensor (9) is associated with said tubular element (2) and is interposed between the intake (3) and the delivery mouth (5) that lies closest to the mouth of the supply, thereby being arranged above the delivery mouths (5).

3. Probe (1) according to claim 2, **characterized by** the fact that said pressure sensor (9) is arranged at a distance from said feeding mouth (3) equal to about 2/3 of the length of said tubular element (2).

4. Probe (1) according to one or more of the preceding claims, **characterized by** the fact that said pressure detecting means (8) comprise a plurality of said pressure sensors (9) arranged mutually spaced apart along the longitudinal extension of said tubular element (2).

5. Probe (1) according to one or more of the preceding claims, **characterized by** the fact that said pressure detecting means (8) comprise electric connection means (10) of said pressure sensor (9) to reading means (11) of the pressure and by the fact that said tubular element (2) comprises at least one connection channel (12), separate from said main channel (7) and adapted to contain said electrical connection means (10).

6. Probe (1) according to claim 5, **characterized by** the fact that said pressure sensor (9) is arranged externally to said connection channel (12).

7. Probe (1) according to one or more of the preceding claims, **characterized by** the fact that it comprises at least an additional pressure sensor associated with said tubular element (2) at said second end stretch (6) to detect the pressure at said delivery mouth (5).

8. Equipment (20) for the forced breathing of a patient, **characterized by** the fact that it comprises at least one probe (1) according to one or more of claims 1 to 7, assisted ventilation means (21) connectable to the respiratory apparatus of a patient and adapted to convey a working gas, at least one control and command unit (22) operatively connected to said ventilation means (21) and to said probe (1) and programmed to regulate the amount of working gas conveyed according to the pressure detected by said pressure sensor (9).

9. Equipment (20) according to claim 8, **characterized by** the fact that said control and command unit (22) is programmed to reduce the amount of working gas delivered by the ventilation means (21) when the pressure detected by said pressure sensor (9) exceeds a predefined value.

## Patentansprüche

1. Nasogastrische Sonde (1), umfassend:
- mindestens ein im Wesentlichen flexibles schlauchförmiges Element (2) mit länglicher Form, das mindestens einen Hauptkanal (7) aufweist, der mit mindestens einer Zuführungsöffnung (3) für Nährsubstanzen oder dergleichen in Verbindung steht, die an einem ersten Endbereich (4) des schlauchförmigen Elements selbst angeordnet ist, und mit mindestens einer Abgabeöffnung (5) für Nährsubstanzen oder dergleichen, die an einem zweiten Endbereich (6) angeordnet ist, der dem ersten Endbereich (4) gegenüberliegt, und dazu bestimmt ist, durch die Speiseröhre in den Magen des Patienten eingeführt zu werden;
- Druckerkennungsmittel (8), die mindestens einen Drucksensor (9) aufweisen, der mit dem schlauchförmigen Element (2) verbunden ist und zwischen der Zuführungsöffnung (3) und der Abgabeöffnung (5) angeordnet ist, wobei der Drucksensor (9) vom Typ eines Druckaufnehmers ist und somit geeignet ist, ein Drucksignal in ein elektrisches Signal umzuwandeln;
**dadurch gekennzeichnet, dass** der Drucksensor (9) außerhalb des Hauptkanals (7) angeordnet ist, so dass er den Durchgang von Flüssigkeiten oder Substanzen in ihm nicht stört, und dass er mit der Außenfläche des schlauchförmigen Elements (2) verbunden ist und ausgebildet ist, einen von der Lunge ausgehenden und durch Zwangsbeatmung verursachten Überdruck zu erfassen.

2. Sonde (1) nach Anspruch 1, **dadurch gekennzeichnet, dass** der Drucksensor (9) mit dem schlauchförmigen Element (2) verbunden ist und zwischen dem Einlass (3) und der Abgabeöffnung (5) angeordnet ist, die am nächsten an der Öffnung der Zuführung liegt, wobei er oberhalb der Abgabeöffnungen (5) angeordnet ist.

3. Sonde (1) nach Anspruch 2, **dadurch gekennzeichnet, dass** der Drucksensor (9) in einem Abstand von der schlauchförmigen Zuführungsöffnung (3) angeordnet ist, der etwa 2/3 der Länge des schlauchförmigen Elements (2) entspricht.

4. Sonde (1) nach einem oder mehreren der vorhergehenden Ansprüche, **dadurch gekennzeichnet, dass** die Druckerkennungsmittel (8) eine Vielzahl der Drucksensoren (9) umfassen, die entlang der Längserstreckung des schlauchförmigen Elements (2) voneinander beabstandet angeordnet sind.

5. Sonde (1) nach einem oder mehreren der vorhergehenden Ansprüche, **dadurch gekennzeichnet, dass** die Druckerkennungsmittel (8) elektrische Verbindungsmittel (10) des Drucksensors (9) zu Drucklesemitteln (11) umfassen und dass das schlauchförmige Element (2) mindestens einen Verbindungskanal (12) aufweist, der von dem Hauptkanal (7) getrennt ist und ausgebildet ist, die elektrischen Verbindungsmittel (10) aufzunehmen.

6. Sonde (1) nach Anspruch 5, **dadurch gekennzeichnet, dass** der Drucksensor (9) außerhalb des Verbindungskanals (12) angeordnet ist.

7. Sonde (1) nach einem oder mehreren der vorhergehenden Ansprüche, **dadurch gekennzeichnet, dass** sie mindestens einen zusätzlichen Drucksensor umfasst, der mit dem schlauchförmigen Element (2) an dem zweiten Endbereich (6) zugeordnet ist, um den Druck an der Abgabeöffnung (5) zu erfassen.

8. Ausrüstung (20) für die Zwangsbeatmung eines Patienten, **dadurch gekennzeichnet, dass** sie mindestens eine Sonde (1) nach einem oder mehreren der Ansprüche 1 bis 7, Mittel zur unterstützten Beatmung (21), die an das Beatmungsgerät eines Patienten angeschlossen werden können und ein Arbeitsgas befördern können, und mindestens eine Steuer- und Befehlseinheit (22) umfasst, die funktionell mit den Beatmungsmitteln (21) und der Sonde (1) verbunden und so programmiert ist, dass sie die Menge des beförderten Arbeitsgases in Abhängigkeit von dem durch den Drucksensor (9) erfassten Druck reguliert.

9. Ausrüstung (20) nach Anspruch 8, **dadurch gekennzeichnet, dass** die Steuer- und Befehlseinheit (22) so programmiert ist, dass sie die von den Beatmungsmitteln (21) abgegebene Arbeitsgasmenge reduziert, wenn der von dem Drucksensor (9) erfasste Druck einen vordefinierten Wert überschreitet.

## Revendications

1. - Sonde nasogastrique (1), comprenant :
- au moins un élément tubulaire sensiblement souple (2) de forme allongée, comportant au moins un canal principal (7) communiquant avec au moins une ouverture d'alimentation (3) de substances nutritives ou analogues, qui est agencée à une première extension d'extrémité (4) de l'élément tubulaire lui-même, et au moins une ouverture de distribution (5) de substances nutritives ou analogues, qui est agencée à une seconde extension d'extrémité (6) opposée à ladite première extension d'extrémité (4) et destinée à être introduite dans l'estomac du patient en passant par l'œsophage ;
- des moyens de détection de pression (8) comprenant au moins un capteur de pression (9) associé audit élément tubulaire (2) et interposé entre ladite ouverture d'alimentation (3) et ladite ouverture de distribution (5), ledit capteur de pression (9) étant du type transducteur de pression, ainsi apte à convertir un signal de pression en un signal électrique ;
**caractérisée par le fait que** ledit capteur de pression (9) est disposé extérieurement audit canal principal (7), de telle sorte qu'il n'interfère pas avec le passage de fluides ou de substances à l'intérieur de celui-ci, et est associé à la surface externe dudit élément tubulaire (2), et est configuré pour détecter la surpression provenant des poumons et causée par une ventilation forcée.

2. - Sonde (1) selon la revendication 1, **caractérisée par le fait que** ledit capteur de pression (9) est associé audit élément tubulaire (2) et est interposé entre l'ouverture d'alimentation (3) et l'ouverture de distribution (5) la plus proche de l'ouverture d'alimentation, étant ainsi disposé au-dessus des ouvertures de distribution (5).

3. - Sonde (1) selon la revendication 2, **caractérisée par le fait que** ledit capteur de pression (9) est disposé à une distance de ladite ouverture d'alimentation (3) égale à environ 2/3 de la longueur dudit élément tubulaire (2).

4. - Sonde (1) selon une ou plusieurs des revendications précédentes, **caractérisée par le fait que** lesdits moyens de détection de pression (8) comprennent une pluralité desdits capteurs de pression (9) disposés espacés les uns des autres le long de l'extension longitudinale dudit élément tubulaire (2).

5. - Sonde (1) selon une ou plusieurs des revendications précédentes, **caractérisée par le fait que** lesdits moyens de détection de pression (8) comprennent des moyens de connexion électrique (10) dudit capteur de pression (9) à des moyens de lecture (11) de la pression et **par le fait que** ledit élément tubulaire (2) comprend au moins un canal de connexion (12), distinct dudit canal principal (7) et apte à contenir lesdits moyens de connexion électrique (10).

6. - Sonde (1) selon la revendication 5, **caractérisée par le fait que** ledit capteur de pression (9) est disposé extérieurement audit canal de connexion (12).

7. - Sonde (1) selon une ou plusieurs des revendications précédentes, **caractérisée par le fait qu'**elle comprend au moins un capteur de pression supplémentaire associé audit élément tubulaire (2) à ladite seconde extension d'extrémité (6) pour détecter la pression à ladite ouverture de distribution (5).

8. - Equipement (20) pour la respiration forcée d'un patient, **caractérisé par le fait qu'**il comprend au moins une sonde (1) selon une ou plusieurs des revendications 1 à 7, des moyens de ventilation assistée (21) aptes à être reliés à l'appareil respiratoire d'un patient et agencés pour transporter un gaz de travail, au moins une unité de contrôle et de commande (22) reliée fonctionnellement auxdits moyens de ventilation (21) et à ladite sonde (1) et programmée pour réguler la quantité de gaz de travail transportée en fonction de la pression détectée par ledit capteur de pression (9).

9. - Equipement (20) selon la revendication 8, **caractérisé par le fait que** ladite unité de contrôle et de commande (22) est programmée pour réduire la quantité de gaz de travail délivrée par les moyens de ventilation (21) lorsque la pression détectée par ledit capteur de pression (9) dépasse une valeur prédéfinie.
